# EUROPÄISCHE PATENTANMELDUNG

(11) **EP 1 226 812 A2**
(43) Veröffentlichungstag der Anmeldung: **31.07.2002**
(21) Anmeldenummer: 02000739.9
(22) Anmeldetag: 12.01.2002
(51) Int. Cl.: A61K 7/06

(54) **Bei Kontakt mit Feuchtigkeit Wärme erzeugende Haarkur**

(30) Priorität: 24.01.2001 DE 10103093
(71) Anmelder: Beiersdorf AG, 20245 Hamburg (DE)
(72) Erfinder: Argembeaux, Horst, 21465 Wentorf (DE); Treu, Jens, Dr., 22844 Norderstedt (DE); Demitz, Michael, 22529 Hamburg (DE)

(57) **Zusammenfassung**

Kosmetische Zubereitungen in Form von Haarkuren, welche dadurch gekennzeichnet sind, daß sie enthalten:
(a) mehr als 70 Gew.-% wenigstens eines Polyalkohols, der bei 25 °C flüssig ist und aus der Gruppe der Polyethylenglycole, der Polypropylenglycole, Glycerin, Diglycerin gewählt wird, unter der Maßgabe, daß die gewählten Substanzen beim Lösen in Wasser Wärme entwickeln (= negative Lösungsenthalpie besitzen)
(b) 0,1 - 2,5 Gew.-% wenigstens einer Substanz, welche eine oder mehrere quaternäre Ammoniumfunktionen enthält,
(c) weniger als 3 Gew.-% Wasser,
(e) gewünschtenfalls eine Ölkomponente,
(f) gewünschtenfalls übliche Hilfs- und Zusatzstoffe.

## Beschreibung

Die vorliegende Erfindung betrifft ein bei Anwendung selbsterwärmendes Haut- und Haarbehandlungsmittel, insbesondere ein Haarkonditioniermittel, das dem menschlichen Haar vor allem ein verbessertes Volumen, erhöhten Glanz und leichte Kämmbarkeit verleiht.

Die vorliegende Erfindung betrifft insbesondere ein Haarbehandlungsmittel mit verbesserten konditionierenden Eigenschaften, insbesondere verbesserter Naß- und Trockenkämmbarkeit, vollem und gleichzeitig lockerem Griff sowie gutem Halt und lebhaftem Glanz, das nach dem Auftragen auf dem Haar verbleiben oder wieder ausgewaschen werden kann.

Die vorliegende Erfindung betrifft schließlich Mittel und Verfahren zur Haarbehandlung, mit denen eine gegenüber herkömmlichen Mitteln und Verfahren verbesserte Konditionierwirkung erreicht wird

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluß des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Ein Ziel der Haarpflege ist es, Kopfhaut und -haar zu schützen und den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für natürliches, gesundes Haar.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Hamsäure, Xanthin, Keratin, Glycogen, Citronensäure, Milchsäure) durch die Haarwäsche herausgelaugt werden.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern. Solche Zubereitungen werden gemeinhin auch als Haarkuren bezeichnet.

Die Eigenschaft der Fülle wird einer Frisur beispielsweise zugeschrieben, wenn das Haar nach der Behandlung nicht flach auf der Kopfhaut aufliegt und gut frisierbar ist.

Die Eigenschaft des Volumens wird einer Frisur beispielsweise zugeschrieben, wenn das Haar nach der Behandlung Fülle und Sprungkraft aufweist.

Die Eigenschaft des Bodys wird einer Frisur beispielsweise zugeschrieben, wenn das Haarvolumen selbst unter äußeren, störenden Einflüssen groß bleibt.

Durch quatemäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Der Stande der Technik ließ es aber an Zubereitungen mangeln, welche dem geschädigten Haar in befriedigender Weise Pflege zukommen ließen. Auch erwiesen sich Zubereitungen, die der Haartracht Fülle geben sollten, oft als unzureichend, zumindest waren sie ungeeignet, als Haarpflegezubereitungen eingesetzt zu werden. Die Haartracht fixierende Zubereitungen des Standes der Technik enthalten beispielsweise in der Regel viskose Bestandteile, welche Gefahr laufen, ein Gefühl der Klebrigkeit zu erwecken, welches oft durch geschickte Formulierung kompensiert werden muß.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, daß sie nicht nur der Pflege des einzelnen Haars dienen, sondern auch das Aussehen einer Frisur insgesamt verbessern, beispielsweise dadurch, daß sie dem Haar mehr Fülle verleihen, die Frisur über einen längeren Zeitraum fixieren oder die Frisierbarkeit verbessern.

Haarkonditioniermittel sind Zusätze zu Haarpflegemitteln, welche die Kämmbarkeit des nassen und des getrockneten Haares verbessern, die statische Aufladung des Haares neutralisieren und zugleich das Aussehen, die Griffigkeit, die Fülle, den Glanz und insgesamt das Frisiervermögen des Haares begünstigen. Die Wirkung eines Haarkonditioniermittels ist weitgehend von dem Aufziehvermögen der Substanz auf die Haaroberfläche abhängig.

Zu den Haarkonditioniermitteln zählen insbesondere die quaternären Ammoniumverbindungen, die verschiedenen Protein-Hydrolysate, femer aber auch verschiedene Fette oder fettähnliche Produkte, z.B. die Silicone und die Silikon-Derivate, Glykole und nach neueren Untersuchungen auch kationische Polymere.

Trotz der Vielzahl der bekannten und vorgeschlagenen Formulierungen sind die bekannten Haarkonditioniermittel nach wie vor verbesserungsfähig, insbesondere ihr Penetrationsvermögen in das Haar und damit ihre Wirksamkeit.

Haarfestigende Mittel und Haarstylingprodukte dienen dazu, die Haare zu festigen, in Form zu bringen und in dieser Form zu halten oder dem Haar mehr Volumen zu geben. Außerdem ist es wünschenswert, daß diese Mittel nach Möglichkeit auch pflegende Wirkungen auf das behandelte Haar besitzen. Derartige Pflegewirkungen sind beispielsweise größeres Feuchthaltevermögen der Haare, antistatischer Effekt, Stärkung des Haares, größerer Glanz usw.. Trockene Haare sind rauh, spröde, glanzlos und verheddern sich leicht an den Enden. Es ist daher erstrebenswert, das Feuchthaltevermögen von Haaren zu verbessern und Austrocknungen vorzubeugen.

Haarbehandlungsmittel, die Metallsalze und Polyole enthalten und beim Mischen mit Wasser Wärme freisetzten sind bekannt. So beschreibt die Schrift EP 586 929 eine Zweikomponentenzubereitungen, bei welcher der eine Teil (A) im wesentlichen wasserfrei ist und ein physiologisch verträgliches Salz enthält, welches beim Lösen in Wasser Wärme entwickelt (= negative Lösungsenthalpie besitzt), und einen zweiten Teil (B), welcher wenigstens einen Polyalkohol enthält, der bei 25 °C flüssig ist und aus der Gruppe Polyethylenglycol, Polypropylenglycol, Glycerin, Diglycerin gewählt wird, unter der Maßgabe, daß die gewählten Substanzen beim Lösen in Wasser Wärme entwickeln (= negative Lösungsenthalpie besitzen).

Diese Zubereitungen zeichnen sich zwar durch eine gute, langanhaltende Wärmeentwicklung aus, haben aber folgende Nachteile:
1. Die kosmetischen Eigenschaften des Produkts sind stark beeinträchtigt. Es leiden der Griff und Glanz des Haares nach der Behandlung mit diesen Produkten.
2. Die Formulierungen sind nur stabil, wenn ihnen größere Mengen an Verdickungsmitteln zugefügt werden. Verdickungsmittel aber beeinträchtigen ihrerseits die Produktleistung.

Diesen Unzulänglichkeiten galt es Abhilfe zu schaffen.

Es wurde nunmehr gefunden, daß man eine ausgezeichnete haarkonditionierende Wirkung erreichen kann, wenn man das Haar mit einer weitgehend wasserfreien Zusammensetzung behandelt, die bei der Anwendung auf dem nassen Haar Wärme entwickelt.

Erfindungsgemäß werden die Aufgaben gelöst durch kosmetische Zubereitungen in Form von Haarkuren, welche dadurch gekennzeichnet sind, daß sie enthalten:
(a) mehr als 70 Gew.-% wenigstens eines Polyalkohols, der bei 25 °C flüssig ist und aus der Gruppe der Polyethylenglycole, der Polypropylenglycole, Glycerin, Diglycerin gewählt wird, unter der Maßgabe, daß die gewählten Substanzen beim Lösen in Wasser Wärme entwickeln (= negative Lösungsenthalpie besitzen)
(b) 0,1 - 2,5 Gew.-% wenigstens einer Substanz, welche eine oder mehrere quaternäre Ammoniumfunktionen enthält,
(c) weniger als 3 Gew.-% Wasser,
(d) weniger als 1 Gew.-% an Elektrolyten,
(e) gewünschtenfalls eine Ölkomponente,
(f) gewünschtenfalls übliche Hilfs- und Zusatzstoffe.

Ohne sich auf eine Theorie festlegen zu wollen, wird angenommen, daß durch die Wärmebehandlung das Penetrationsvermögen in das Haar wesentlich erhöht wird, wobei die wärmegenerierenden Inhaltsstoffe selbst als konditionierende Substanzen wirken und/oder diese Wirkung durch zusätzlich mitverwendete, an sich bekannte haarpflegende Mittel erzielt wird, wobei die Wirkung der letzteren Substanzen durch die Anwendung der Erfindung in synergistischer Weise verstärkt wird.

Ein großer Vorteil der erfindungsgemäßen Zubereitung ist deren einfache Handhabung bei der Anwendung. Im Gegensatze zu den bekannten Mehrkomponentesystemen kommt hier nur eine Komponente zum Einsatz, wodurch der Anwender erhebliche Erleichterung erfährt.

Erfindungsgemäß ist auch ein kosmetisches Verfahren zur Pflege des Haupthaares und der Kopfhaut, dadurch gekennzeichnet, daß eine wirksame Menge einer Zubereitung, welche enthält:
(a) mehr als 70 Gew.-% wenigstens eines Polyalkohols, der bei 25 °C flüssig ist und aus der Gruppe der Polyethylenglycole, der Polypropylenglycole, Glycerin, Diglycerin gewählt wird, unter der Maßgabe, daß die gewählten Substanzen beim Lösen in Wasser Wärme entwickeln (= negative Lösungsenthalpie besitzen)
(b) 0,1 - 2,5 Gew.-% wenigstens einer Substanz, welche eine oder mehrere quaternäre Ammoniumfunktionen enthält,
(c) weniger als 3 Gew.-% Wasser,
(e) gewünschtenfalls eine Ölkomponente,
(f) gewünschtenfalls übliche Hilfs- und Zusatzstoffe,
auf feuchtes Haar aufgebracht und dort verteilt wird, einen ausreichenden Zeitraum auf dem Haar verbleibt und nach Ablauf dieses Zeitraumes mit Hilfe von Wasser wieder aus dem Haar ausgespült wird.

Das erfindungsgemäße Mittel wird angewandt, indem eine für die gewünschte Pflegewirkung ausreichende Menge in oder auf dem feuchten oder handtuchgetrockneten Haar oder nach der Haarwäsche in oder auf dem Haar verteilt wird. Die anzuwendende Menge hängt von der Haarfülle ab und beträgt typischerweise 5 bis 30g.

Das Mittel wird nach einem Zeitraum von typischerweise 5 bis 30 Minuten ausgespült. Anschließend wird das Haar gegebenenfalls durchgekämmt oder zur Frisur geformt und gegebenenfalls getrocknet.

Als wärmegenerierendes Agenz der erfindungsgemäßen Zubereitungen kommen Polyalkohole zum Einsatz. Bevorzugt werden dabei solche mit einem Molekulargewicht zwischen 200 und 600 g/mol, beispielsweise Polyethylenglycol(200) (= PEG-4), Polyethylenglycol(200) (= PEG-4), Polyethylenglycol(300) (= PEG-6), Polyethylenglycol(400) (= PEG-8), Polyethylenglycol(1000) (= PEG-5) und Polyethylenglycol(1200) (= PEG-6), wobei der bevorzugte Polyalkohol das PEG-8 ist.

Beim Kontakt mit dem nassen Haar wird durch das Lösen der Polyalkohole, bevorzugt das PEG-8, in Wasser Wärmeenergie freigesetzt. Darüber hinaus speichern die Polyalkohole die Wärme, was zu einer langanhaltenden Temperaturerhöhung während der Behandlung führt.

Zusätzlich zeigen die Polyalkohole als solche bereits haarpflegende Eigenschaften. Es ist aber zweckmäßig und vorteilhaft, weitere an sich bekannte haarkonditionierende Wirkstoffe mitzuverwenden.

Quatemäre Ammonium-Verbindungen sind eine wichtige Gruppe der speziellen Wirkstoffe, die zur Herstellung von Haarpflegemitteln verwendet werden. Haarpflegemittel, insbesondere Haarkuren, erhalten wesentliche Eigenschaften wie Verbesserung von Kämmbarkeit sowie Griff und Verhinderung statischer Aufladung der Haare vornehmlich durch den Einsatz quaternärer Ammonium-Verbindungen.

Die Eigenschaften quaternärer Ammonium-Verbindungen werden durch die kationische Gruppe einerseits und durch die Art der lipophilen Reste dieser Gruppe andererseits bestimmt. Geeignet sind z.B. die Verbindungen, in denen ein bis zwei Reste längerkettige Alkyl-Gruppen, wie Lauryl-, Cetyl- oder Stearyl-Gruppen, und die verbliebenen Reste Methyl-Gruppen sind. Produkte dieses Typs werden vorzugsweise als Chloride, Bromide und Methosulfate eingesetzt.

Geeignete Polymere mit kationischen Gruppen enthalten vorzugsweise quatemäre Amingruppen. Die kationischen Polymere können Homo- oder Copolymere sein, wobei die quaternären Stickstoffgruppen entweder in der Polymerkette oder vorzugsweise als Substituent an einem oder mehreren Monomeren enthalten sind. Die Ammoniumgruppen enthaltenden Monomere können mit den oben genannten nicht aminsubstituierten Monomeren copolymerisiert sein. Geeignete ammoniumsubstituierte Vinylmonomere sind zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z. B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C₁- bis C₇-Alkylgruppen, besonders bevorzugt C₁- bis C₃-Alkylgruppen.

Geeignete kationische Polymere sind beispielsweise kationische Cellulosederivate (z.B. Polymer JR 400® von Amerchol), kationische Stärke, Copolymere von Diallylammoniumsalzen und Acrylamiden, quatemisierte VinylpyrrolidonNinyl-imadazol-Polymere (z.B. Luviquat® von der BASF), Kondensationsprodukte von Polyglycolen und Aminen, quaternisierte Kollagenpolypeptide (z.B. Lamequat® L von Grünau-Henkel), quaternisierte Weizenpolypeptide, Polyethylenimin, kationische Silikonpolymere, Copolymere der Adipinsäure mit Dimethylaminohydroxypropyldietylentriamin, Copolymere der Acrylsäure mit Dimethyldiallylamoniumchlorid (z.B. Merquat® 550 von Chemviron), Polyaminopolyamide, kationische Chitinderivate, kationischer Guar-Gum (z.B. Jaguar® CBS von Hoechst Celanese), quaternisierte Ammoniumsalz-Polymere (z.B. Mirapol® AD-1 von Miranol) sowie kationische Biopolymere wie z.B. Chitosan (mittleres Molekulargewicht von 50.000 bis 2.000.000 g/mol [bestimmt mittels Gelpermeationschromatographie] und einem Deacylierungsgrad von 10 bis 99% [bestimmt mittels ¹H-NMR]).

Als filmbildendes Polymer mit wenigstens teilweise quaternisierten Stickstoffgruppen eigenen sich bevorzugt solche, welche gewählt werden aus der Gruppe der Substanzen, welche nach der INCI-Nomenklatur (International Nomenclature Cosmetic Ingredient) den Namen "Polyquatemium" tragen, beispielsweise:
- Polyquaternium-2: (Chemical Abstracts-Nr. 63451-27-4, z.B. Mirapol® A-15)
- Polyquaternium-5: (Copolymeres aus dem Acrylamid und dem β-Methacryloxyethyltrimethylammoniummethosulfat, CAS-Nr. 26006-22-4)
- Polyquaternium-6: (Homopolymer des N,N-Dimethyl-N-2-propenyl-2-propen-1-aminiumchlorids, CAS-Nr. 26062-79-3, z.B. Merquat® 100
- Polyquatemium-7: N,N-Dimethyl-N-2-propenyl-2-propen-1-aminiumchlorid, Polymeres mit 2-Propenamid, CAS-Nr. 26590-05-6, z.B. Merquat® S
- Polyquaternium-10: Quaternäres Ammoniumsalz der Hydroxyethylcellulose, CAS-Nr. 53568-66-4, 55353-19-0, 54351-50-7, 68610-92-4, 81859-24-7, z.B. Celquat® SC-230M,
- Polyquaternium-11: Vinylpyrrolidon/dimethylaminoethyl-Methacrylat-Copolymer/Diethylsulfat-Reaktionsprodukt, CAS-Nr. 53633-54-8, z.B. Gafquat® 755N
- Polyquaternium-16: Vinylpyrrolidon/vinylimidazoliniummethochlorid-Copolymer, CAS-Nr. 29297-55-0, z.B. Luviquat® HM 552
- Polyquaternium-17: CAS-Nr. 90624-75-2, z.B. Mirapol® AD-1
- Polyquatemium-19: Quaternisierter wasserlöslicher Polyvinylalkohol
- Polyquaternium-20: in Wasser dispergierbarer quaternisierter Polyvinyloctadecylether
- Polyquaternium-21: Polysiloxan-polydimethyl-dimethylammoniumacetat-Copolymeres, z.B. Abil® B 9905
- Polyquaternium-22: Dimethyldiallylammoniumchlorid/Acrylsäure-Copolymer, CAS-Nr. 53694-7-0, z.B. Merquat® 280
- Polyquatemium-24: Polymeres quatemäres Ammoniumsalz der Hydroxyethylcellulose, Reaktionsprodukt mit einem mit Lauryldimethylammonium substituierten Epoxid, CAS-Nr. 107987-23-5, z.B. Quatrisoft® LM-200
- Polyquaternium-28: Vinylpyrrolidon/Methacrylamidopropyltrimethylammoniumchlorid-Copolymer, z.B. Gafquat® HS-100
- Polyquaternium-29: z.B. Lexquat® CH
- Polyquaternium-31: CAS-Nr. 136505-02-7, z.B. Hypan® QT 100
- Polyquaternium-32: N,N,N-trimethyl-2-[(2-methyl-1-oxo-2-propenyl)oxy]-Ethanaminiumchlorid, polymer mit 2-Propenamid, CAS-Nr. 35429-19-7
- Polyquaternium-37: CAS-Nr. 26161-33-1

Quaternäre Ammoniumverbindungen können insbesondere vorteilhaft gewählt werden aus der Gruppe Behenalkoniumchlorid, (3-Behenoyloxy-2-hydroxypropyl)-trimethylammoniumchlorid, Behentrimoniumchlorid, Behenyltrimethylammoniummethosulfat, Benzyldimethylstearylammoniumchlorid, Benzyldocosyldimethylammoniumchlorid, Bis(N-Hydroxyethyl-2-oleylimidazoliniumchlorid)-polyethylenglykol(600), Bis(Talg[hydriert]alkyl)dimethylmethylsulfat, quaternisiert, Cetearalkoniumbromid, Cetrimoniumbromid, Cetrimoniumchlorid, Cetrimoniumtosylat, Cetyldimethylbenzylammoniumchlorid, Cetyldimethylethylammoniumbromid, Cetylethylmorpholiniumethosulfat, Cetylpyridiniumbromid, Cetyl-Stearyldimethylbenzylammoniumbromid, N-Cetyltrimethylammoniumbromid, Cocamidopropyldimethylacetamidoammoniumchlorid, Cocosnußtrimethylammoniumchlorid, Cocotrimoniumchlorid, Cocoylbenzylhydroxyethylimidazoliniumchlorid, Cocoylhydroxyethylimidazolin, Decyldimethyloctylammoniumchlorid, Dicocodimethylammoniumchlorid, Didecyldimethylammoniumchlorid, Dimethyl(hydrierte Talgfettsäuren)ammoniumchlorid, Dimethylbenzyltalgammoniumchlorid, Dimethylcyclohexylcetylammoniumstearat, Dimethyldialkylammoniumchlorid, Dimethyldi(Talg, hydriert)ammoniumchlorid, Dimethyldilaurylammoniumchlorid, Dimethyldi(hydriert. Talg)ammoniumsalz, Dimethyldistearylammoniumchlorid, Dimethylditalgammoniumchlorid, Dimethylhydroxyethylcetylammoniumchlorid, Dipalmethylhydroxyethylmoniummethosulfat(e), Distearyldimethylammoniumchlorid, Dodecylbenzyltriethanolammoniumchlorid, Dodecylbenzyltrimethylammoniumchlorid, Dodecyl-3,4-dichlorbenzyldimethylammoniumbromid, Dodecyldimethylammoniumchlorid, Dodecyldimethylethylbenzylammoniumchlorid, Dodecyldimethyl- (2- phenoxyethyl)ammoniumbromid, Dodecyl-di(b-oxyethyl)benzylammoniumchlorid, Dodecylxylylbis(trimethylammoniumchlorid), hydriertes Talgammoniumchlorid, Hydroxyanthrachinonaminopropyl-4-methylmorpholiniummethosulfat, ß-Hydroxydodecyldimethylbenzylammoniumchlorid, Hydroxyethylcellulose-Polymeres, (Hydroxyethyl)dimethyl(3-[Nerzöl]amidopropyl)-chlorid, C₁₄₋₂₀-Isoalkylamidopropylethyldimonium Ethosulfate, C₁₈₋₂₂-Isoalkylamidopropylethyldimoniumethosulfat, Isostearylbenzylimidoniumchlorid, Isostearylethylimidoniumethosulfat, Lapyriumchlorid (N-(Laurylcolaminoformylmethyl)pyridiniumchlorid), Laurtrimoniumchlorid, Laurylalkoniumchlorid, Laurylisochinolinbromid, Laurylpyridiniumchlorid, Laurylpyridiniumjodid, Methylbenzethoniumchlorid, Methylen-bis(talgacetamido)diammoniumchlorid, Myristyldimethylbenzylammoniumchlorid, Myristyltrimethylammoniumbromid, Octyldecyldimethylammoniumchlorid, Olealkoniumchlorid, Oleyldimethylbenzylammoniumchlorid, N,N,N',N',N'-Pentamethyl-N-talgalkyl-1,3-propandiammoniumdichlorid, Pentaoxyethylenstearylammoniumbromid, β-Phenoxyethyldimethyldodecylammoniumbromid, Polyethylenglykol(5)stearylammoniumlactat, Polyoxypropylen(9)methyldiethylammoniumchlorid, Polyoxypropylen-(25)-methyldiethylammoniumchlorid, Polyoxypropylen(40)methyldiethylammoniumchlorid, Rübölamidopropylbenzyldimoniumchlorid, Rübölamidopropylethyldimoniumethosulfat, Sojaamidopropylbenzyldimoniumchlorid, Sojaamidopropylethyldimoniumethosulfat, N-Soja-N-ethylmorpholiniumethosulfat, Sojatrimethylammoniumchlorid, Stearalkoniumchlorid, N-(Stearoylcolaminoformylmethyl)-pyridiniumchlorid, Stearyldimethylbenzylammoniumchlorid, Stearylpolyglykolphosphatdimoniumchlorid, Stearyltrimethylammoniumchlorid, Talgalkoniumchlorid, Talgtrimethylammoniumchlorid, Tallölbenzylhydroxyethylimidazoliniumchlorid, Tetramethylammoniumchlorid, Wollwachs-isostearamidopropylethyldimethylethosulfat.

Weitere, gegebenenfalls vorteilhaft eingesetzte Polymere mit sauren Gruppen sind amphotere Polymere, welche neben den sauren Gruppen als weitere funktionelle Gruppen basische oder kationische Gruppen, insbesondere primäre, sekundäre, tertiäre oder quatemäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren bestehend aus Acrylsäure, Methacrylsäure oder deren Ester, wie sie zum Beispiel unter dem Handelsnamen Amphomer® oder Amphomer® LV-71 der Firma NATIONAL STARCH, USA erhältlich sind. Weitere Beispiele sind Copolymere von Acrylsäure, Methylacrylat und Methacrylamidopropyltrimethylammoniumchlorid (INCI- Bezeichnung: Polyquaternium-47), wie sie beispielsweise von der Firma Calgon unter der Handelsbezeichnung Merquat® 2001 vertrieben werden, Copolymere aus Acrylamidopropyltrimethylammoniumchlorid und Acrylaten, wie sie beispielsweise von der Firma Stockhausen unter der Handelsbezeichnung W 37194 erhältlich sind oder Copolymere aus Acrylamid, Acrylamidopropyltrimethyl- ammoniumchlorid, 2-Amidopropylacrylamidsulfonat und Dimethylaminopropylamin (INCl-Bezeichnung: Polyquaternium-43), wie sie beispielsweise von der Firma Societe Francaise Hoechst unter der Handelsbezeichnung Bozequat® 4000 vertrieben werden. Geeignet sind auch Polymere mit Betaingruppen tragenden Monomeren wie z. B. Copolymere aus Methacryloylethylbetain und zwei oder mehr Monomeren von Acrylsäure oder deren einfachen Estem, bekannt unter der INCI-Bezeichnung Methacryloyl Ethyl Betaine/Acrylates Copolymer. Geeignet sind auch die zwitterionischen amphoteren Polymere, die in der JP 10-29919 oder in der JP10-25344 beschrieben sind.

Die Haarpflegemittel und Zubereitungen, die erfindungsgemäße Wirkstoffe enthalten, sind topische Zubereitungen. Diese können wie üblich zusammengesetzt sein und zur Behandlung und der Pflege der Kopfhaut und/oder der Haare oder als Lichtschutzpräparat dienen. Zur Anwendung werden die erfindungsgemäßen Zubereitungen in der für Kosmetika und Haarpflegemittel üblichen Weise auf die Kopfhaut und die Haare in ausreichender Menge aufgebracht.

Die Mittel gemäß der Erfindung können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels.

Als Treibmittel für aus Aerosolbehältem versprühbare kosmetische oder dermatologische Zubereitungen im Sinne der vorliegenden Erfindung, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Vorteilhaft enthalten erfindungsgemäße Zubereitungen neben einem wirksamen Gehalt an erfindungsgemäßen Wirkstoffen femer übliche Wirk-, Inhalts-, Zusatz- und/oder Hilfsstoffe.

Die Erfindung kann vorteilhaft eine oder mehrere Ölkomponenten enthalten, die vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölkomponenten werden vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, O-leylerucat, Erucyloleat, Erucylerucat sowie synthetische, halbsynthetische und natürliche Gemische solcher Ester, z.B. Jojobaöl.

Ferner können die Ölkomponenten vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft werden die Ölkomponenten gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft können die Ölkomponenten femer einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind femer Mischungen aus Cyclomethicon und Isotridecylisononanoat, aus Cyclomethicon und 2-Ethylhexylisostearat.

Vorteilhafte weitere Grundstoffe für Haarpflegemittel sind z.B. Fettalkohole, Wachse, Paraffine, Vaseline, Paraffinöl und Lösemittel.

Fettalkohole sind z.B. gerad- oder verzweigtkettige aliphatische einwertige Alkohole mit 6 - 22 C-Atomen im Molekül. In der Kosmetik werden vorzugsweise geradkettige Fettalkohole mit einer Kettenlänge von 12 - 18 C-Atomen verwendet. Diese Fettalkohole sind weiche, farblose Massen, praktisch ungiftig und gut hautverträglich. Fettalkohole werden bevorzugt zur Herstellung von erfindungsgemäßen Haarkuren verwendet, wobei dem Cetylalkohol und dem Stearylalkohol besondere Bedeutung zukommt.

Wachse sind Fettsäureester, die in tierischen und pflanzlichen Produkten vorkommen, aber auch synthetisch hergestellt werden können. Das wohl bekannteste natürlich vorkommende Wachs ist das Bienenwachs, das als Hauptbestandteile Cerin und Myricin enthält. Wachs ist jedoch ein Oberbegriff für eine Reihe natürlich oder künstlich gewonnener Stoffe, die in der Regel halbfeste, weiße, geruchlose und in Wasser unlösliche Massen darstellen.

Paraffine im kosmetischen Sinn sind weiße, geruchlose Massen aus geradkettigen hochmolekularen Kohlenwasserstoffen. Wegen ihrer den der Wachse vergleichbaren Eigenschaften werden sie auch oft als Erdölwachse bezeichnet.

Vaseline ist ein Gemisch von verzweigtkettigen Paraffinen mit geringem Anteil an zyklischen Paraffinen. Es ist eine weiche, transparente und in Wasser unlösliche Masse mit geringem Eigengeruch, die bei der Aufbereitung des Erdöls anfällt.

Paraffinöl ist ein Gemisch gesättigter flüssiger Kohlenwasserstoffe. Es ist unlöslich in Wasser, aber mischbar mit Fettalkoholen und Wachsen. Es wird als Zusatz in Haarpflegemitteln zur Konsistenzregulierung verwendet.

Hilfsstoffe können verwendet werden, um bestimmte Eigenschaften der Haarpflegemittel, z.B. Konsistenz, Temperatur- und Lichtstabilität, Aussehen und Geruch, zu verbessern sowie deren Herstellung zu erleichtem. Zugesetzt werden z.B. nach Bedarf:
- Emulgatoren, um die Grenzflächenspannung zwischen zwei an sich nicht mischbaren Phasen so weit herabzusetzen, daß deren feine Vermischung möglich wird,
- Verdickungsmittel, um die Stabilität von Emulsionen zu erhöhen und deren Viskosität einzustellen,
- UV-Absorber, um die Lichtstabilität der in Haarpflegemitteln enthaltenen Farbstoffe und anderer lichtempfindlicher Komponenten zu verbessern. Daneben dienen sie dem Schutz des Haares vor Lichteinflüssen.
- Konservierungsmittel, um mikrobieller Zersetzung vorzubeugen.
- Antioxidantien, um Geruchsveränderungen, die durch Oxidationsvorgänge hervorgerufen werden können, zu verhindern.
- Farbstoffe, um Haarpflegemitteln ein ansprechendes Aussehen zu verleihen.
- Parfümöle, um Haarpflegemitteln einen angenehmen Duft zu verleihen und Nebengerüche der Rohstoffe zu überdecken.

Die Menge der Grundstoffe beträgt beispielsweise 85 bis 99,999 Gew.-%, vorzugsweise 90 bis 99,99 Gew.-%, jeweils bezogen auf das Gesamtgewicht der Zubereitung.

Erfindungsgemäße Zubereitungen enthalten vorteilhaft einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel

   R-O-(-CH₂-CH₂-O-)ₙ-R',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH

   und n eine Zahl von 5 bis 30 darstellen,
- der Polyoxyethylensorbitolfettsäureester,
- der Alkylethersulfate der allgemeinen Formel

   R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19-), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat
Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21 )glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Es kann auch gegebenenfalls vorteilhaft sein, in erfindungsgemäßen Zubereitungen solche oberflächenaktive Substanzen einzusetzen, die als waschaktive Tenside bekannt sind. Bevorzugt sind kationische und nichtionische bzw. amphotere Tenside.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfatoder Sulfonatgruppen auf. In wäßriger Lösung bilden sie im sauren oder neutralen Milieu negativ geladene organische lonen. Kationische Tenside sind beinahe ausschließlich durch das Vorhandensein einer quartemären Ammoniumgruppe gekennzeichnet. In wäßriger Lösung bilden sie im sauren oder neutralen Milieu positiv geladene organische lonen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

RNH₂⁺CH₂CH₂COOH X⁻ (bei pH=2) X⁻= beliebiges Anion, z.B. Cl⁻

RNH₂⁺CH₂CH₂COO⁻ (bei pH=7)

RNHCH₂CH₂COO⁻ B⁺(bei pH=12) B⁺ = beliebiges Kation, z.B. Na⁺

Typisch für nicht-ionische Tenside sind Polyether-Ketten. Nicht-ionische Tenside bilden in wäßrigem Medium keine lonen.

### A. Anionische Tenside

Gegebenenfalls vorteilhaft zu verwendende anionische Tenside sind Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Gegebenenfalls vorteilhaft zu verwendende kationische Tenside sind
1. Alkylamine,
2. Alkylimidazole,
3. Ethoxylierte Amine und
4. Quatemäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quatemäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quatemären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryloder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Gegebenenfalls vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Gegebenenfalls vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Monomere oder polymere quaternäre Ammonium-Verbindungen werden vielfach in Haarspülungen und Haarkuren z.B. in Konzentrationen von 0,5 - 5 Gew.-% eingesetzt. Dazu gehören Cetrimoniumchlorid wie es unter der Bezeichnung Dehyquart® A von der Gesellschaft Henkel angeboten wird oder Distearoylethylhydroxyethylmoniummethosulfat wie es unter der Bezeichnung Dehyquart® F 75 von der Gesellschaft Henkel angeboten wird.

Die vorstehenden Prozentangaben beziehen sich auf das Gesamtgewicht der Zubereitungen.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, Antioxidantien, Stabilisatoren, pH-Wert-Regulatoren, Konsistenzgeber, Bakterizide, Desodorantien, antimikrobielle Stoffe, Antistatika, UV-Absorber, Komplexierungsund Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Erfindungsgemäß können als weitere Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,000.001 bis 2 Gew.-%, vorzugsweise 0,001 bis 1 Gew.-%, jeweils bezogen auf das Gesamtgewicht der Zubereitung.

Vorteilhaft werden weitere Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesterylund Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die folgenden Beispiele verdeutlichen die Erfindung. Die Mengenangaben in den Beispielen sind Gew.-%.

| **Beispiele einer selbsterwärmenden Haarkur:** | | | | | | | |
|---|---|---|---|---|---|---|---|
| | **I** | **II** | **III** | **IV** | **V** | **VI** | **VI** |
| Glycerylstearat | - | 4,0 | - | - | - | - | - |
| Citronensäure | - | - | - | - | - | 0,1 | 0,1 |
| Myristylalcohol | - | 4,0 | - | - | - | - | - |
| Cetylstearylalcohol | 1,0 | 1,0 | 6,0 | 6,0 | 5,0 | 5,0 | 5,0 |
| Ceteareth-20 | 2,0 | - | 4,0 | - | - | - | - |
| PEG-8 | 92,2 | 87,8 | 86,6 | 86,6 | 86,6 | 86,5 | 86,5 |
| Bis-Diglycerypolyacyladipat | - | - | - | - | - | 4,0 | 4,0 |
| Laureth-23 | - | - | - | - | 4,0 | - | - |
| PEG-200 Hydriertes Glycerylpalmat | 2,0 | - | - | - | - | - | - |
| Behentrimoniumchlorid | 1,0 | 1,0 | 1,0 | 1,0 | - | 2,0 | - |
| Oleth-20 | - | - | - | 4,0 | - | - | - |
| PEG-14 M | - | 0,4 | 0,6 | 0,6 | 0,6 | 0,6 | 0,6 |
| Dimethiconcopolyol | 1,0 | 1,0 | 1,0 | 1,0 | 1,0 | 1,0 | 1,0 |
| Quaternium-87 | - | - | - | - | - | - | 2,0 |
| Palmitamidopropyltrimoniumchlorid | - | - | - | - | 2,0 | - | - |
| Parfum | 0,8 | 0,8 | 0,8 | 0,8 | 0,8 | 0,8 | 0,8 |

## Patentansprüche

1. Kosmetische Zubereitungen in Form von Haarkuren, welche **dadurch gekennzeichnet sind, daß** sie enthalten:
(a) mehr als 70 Gew.-% wenigstens eines Polyalkohols, der bei 25 °C flüssig ist und aus der Gruppe der Polyethylenglycole, der Polypropylenglycole, Glycerin, Diglycerin gewählt wird, unter der Maßgabe, daß die gewählten Substanzen beim Lösen in Wasser Wärme entwickeln (= negative Lösungsenthalpie besitzen)
(b) 0,1 - 2,5 Gew.-% wenigstens einer Substanz, welche eine oder mehrere quatemäre Ammoniumfunktionen enthält,
(c) weniger als 3 Gew.-% Wasser,
(e) gewünschtenfalls eine Ölkomponente,
(f) gewünschtenfalls übliche Hilfs- und Zusatzstoffe.

2. Zubereitungen nach Anspruch 1, **dadurch gekennzeichnet, daß** der oder die Polyalkohole gewählt werden aus der Gruppe der Substanzen mit einem Molekulargewicht zwischen 200 und 600 g/mol, beispielsweise Polyethylenglycol(200) (= PEG-4), Polyethylenglycol(200) (= PEG-4), Polyethylenglycol(300) (= PEG-6), Polyethylenglycol(400) (= PEG-8), Polyethylenglycol(1000) (= PEG-5) und Polyethylenglycol(1200) (= PEG-6).

3. Zubereitungen nach Anspruch 1, **dadurch gekennzeichnet, daß** die Substanz oder die Substanzen, welche eine oder mehrere quaternäre Ammoniumfunktionen enthalten, gewählt werden aus der Gruppe Behenalkoniumchlorid, (3-Behenoyloxy-2-hydroxypropyl)-trimethylammoniumchlorid, Behentrimoniumchlorid, Behenyltrimethylammoniummethosulfat, Benzyldimethylstearylammoniumchlorid, Benzyldocosyldimethylammoniumchlorid, Bis(N-Hydroxyethyl-2-oleylimidazoliniumchlorid)-polyethylenglykol(600), Bis(Talg[hydriert]alkyl)dimethylmethylsulfat, quaternisiert, Cetearalkoniumbromid, Cetrimoniumbromid, Cetrimoniumchlorid, Cetrimoniumtosylat, Cetyldimethylbenzylammoniumchlorid, Cetyldimethylethylammoniumbromid, Cetylethylmorpholiniumethosulfat, Cetylpyridiniumbromid, Cetyl-Stearyldimethylbenzylammoniumbromid, N-Cetyltrimethylammoniumbromid, Cocamidopropyldimethylacetamidoammoniumchlorid, Cocosnußtrimethylammoniumchlorid, Cocotrimoniumchlorid, Cocoylbenzylhydroxyethylimidazoliniumchlorid, Cocoylhydroxyethylimidazolin, Decyldimethyloctylammoniumchlorid, Dicocodimethylammoniumchlorid, Didecyldimethylammoniumchlorid, Dimethyl(hydrierte Talgfettsäuren)ammoniumchlorid, Dimethylbenzyltalgammoniumchlorid, Dimethylcyclohexylcetylammoniumstearat, Dimethyldialkylammoniumchlorid, Dimethyldi(Talg, hydriert)ammoniumchlorid, Dimethyldilaurylammoniumchlorid, Dimethyldi(hydriert. Talg)ammoniumsalz, Dimethyldistearylammoniumchlorid, Dimethylditalgammoniumchlorid, Dimethylhydroxyethylcetylammoniumchlorid, Dipalmethylhydroxyethylmoniummethosulfat(e), Distearyldimethylammoniumchlorid, Dodecylbenzyltriethanolammoniumchlorid, Dodecylbenzyltrimethylammoniumchlorid, Dodecyl-3,4-dichlorbenzyldimethylammoniumbromid, Dodecyldimethylammoniumchlorid, Dodecyldimethylethylbenzylammoniumchlorid, Dodecyldimethyl- (2-phenoxyethyl)ammoniumbromid, Dodecyl-di(b-oxyethyl)benzylammoniumchlorid, Dodecylxylylbis(trimethylammoniumchlorid), hydriertes Talgammoniumchlorid, Hydroxyanthrachinonaminopropyl-4-methylmorpholiniummethosulfat, β-Hydroxydodecyldimethylbenzylammoniumchlorid, Hydroxyethylcellulose-Polymeres, (Hydroxyethyl)dimethyl(3-[Nerzöl]amidopropyl)-chlorid, C₁₄₋₂₀-Isoalkylamidopropylethyldimonium Ethosulfate, C₁₈₋₂₂-Isoalkylamidopropylethyldimoniumethosulfat, Isostearylbenzylimidoniumchlorid, Isostearylethylimidoniumethosulfat, Lapyriumchlorid (N-(Laurylcolaminoformylmethyl)pyridiniumchlorid), Laurtrimoniumchlorid, Laurylalkoniumchlorid, Laurylisochinolinbromid, Laurylpyridiniumchlorid, Laurylpyridiniumjodid, Methylbenzethoniumchlorid, Methylen-bis(talgacetamido)diammoniumchlorid, Myristyldimethylbenzylammoniumchlorid, Myristyltrimethylammoniumbromid, Octyldecyldimethylammoniumchlorid, Oleyldimethylbenzylammoniumchlorid, N, N, N', N', N'-Pentamethyl-N-talgalkyl-1,3-propandiammoniumdichlorid, Olealkoniumchlorid,Pentaoxyethylenstearylammoniumbromid, β-Phenoxyethyldimethyldodecylammoniumbromid, Polyethylenglykol(5)-stearylammoniumlactat, Polyoxypropylen(9)methyldiethylammoniumchlorid, Polyoxypropylen-(25)-methyldiethylammoniumchlorid, Polyoxypropylen-(40)methyldiethylammoniumchlorid, Rübölamidopropylbenzyldimoniumchlorid, Rübölamidopropylethyldimoniumethosulfat, Sojaamidopropylbenzyldimoniumchlorid, Sojaamidopropylethyldimoniumethosulfat, N-Soja-N-ethylmorpholiniumethosulfat, Sojatrimethylammoniumchlorid, Stearalkoniumchlorid, N-(Stearoylcolaminoformylmethyl)-pyridiniumchlorid, Stearyldimethylbenzylammoniumchlorid, Stearylpolyglykolphosphatdimoniumchlorid, Stearyltrimethylammoniumchlorid, Talgalkoniumchlorid, Talgtrimethylammoniumchlorid, Tallölbenzylhydroxyethylimidazoliniumchlorid, Tetramethylammoniumchlorid, Wollwachs-isostearamidopropylethyldimethylethosulfat.

4. Kosmetisches Verfahren zur Pflege des Haupthaares und der Kopfhaut, **dadurch gekennzeichnet, daß** eine wirksame Menge einer Zubereitung, welche enthält:
(a) mehr als 70 Gew.-% wenigstens eines Polyalkohols, der bei 25 °C flüssig ist und aus der Gruppe der Polyethylenglycole, der Polypropylenglycole, Glycerin, Diglycerin gewählt wird, unter der Maßgabe, daß die gewählten Substanzen beim Lösen in Wasser Wärme entwickeln (= negative Lösungsenthalpie besitzen)
(b) 0,1 - 2,5 Gew.-% wenigstens einer Substanz, welche eine oder mehrere quaternäre Ammoniumfunktionen enthält,
(c) weniger als 3 Gew.-% Wasser,
(e) gewünschtenfalls eine Ölkomponente,
(f) gewünschtenfalls übliche Hilfs- und Zusatzstoffe,
auf feuchtes Haar aufgebracht und dort verteilt wird, einen ausreichenden Zeitraum auf dem Haar verbleibt und nach Ablauf dieses Zeitraumes mit Hilfe von Wasser wieder aus dem Haar ausgespült wird.
